# EUROPEAN PATENT APPLICATION

(11) **EP 1 598 001 A1**
(43) Date of publication of application: **23.11.2005**
(21) Application number: 04713910.0
(22) Date of filing: 24.02.2004
(51) Int. Cl.: A61B 1/267

(54) **LUMINOUS OPTICAL LARYNGOSCOPE COMPRISING BUILT-IN FLUID-EXTRACTION DEVICE**

(30) Priority: 24.02.2003 ES 200300441
(71) Applicant: SCB, S.A., 48930 Getxo, Vizcaya (ES)
(72) Inventor: ACHA GANDARIAS, Pedro, 48990 Getxo (ES)
(74) Representative: Primo de Rivera y Urquijo, Jose A.
(86) International application number: PCT/ES2004/000085
(87) International publication number: WO 2004/073510

(57) **Abstract**

The invention relates to an optical light laryngoscope with a built-in fluid extraction device, of the type comprising two independent conduits. One of the aforementioned conduits is equipped with optical means for viewing the inside of the larynx while the other conduit is used for introducing the endotracheal tube into the larynx. The inventive laryngoscope also comprises a built-in fluid extraction device which is used to withdraw fluids from the mouth in order to optimize the view when the endotracheal tube is being inserted into the patient in a convenient, safe and hygienic manner.

## Description

### OBJECT OF THE INVENTION

The optical light laryngoscope with a built-in fluid extraction device object of the present invention consists of an optical light laryngoscope of the type made up of two independent conduits, one provided with optical means for visualizing the interior of the larynx and the other one for introducing the endotracheal tube in the larynx, in addition to a built-in fluid extraction device in the laryngoscope, the intention of which is to withdraw fluids existing in the mouth in order to achieve optimal vision during the introduction of the endotracheal tube in the patient in a comfortable, simple and hygienic manner.

### DESCRIPTION OF THE STATE OF THE ART

Different laryngoscopes are known in the state of the art, but the improvements implied from the optical light laryngoscope disclosed in European patent application EP-A-1285623 of the same applicant are particularly relevant. The device disclosed in said application allows introducing the endotracheal tube into the patient with fewer risks for the patient due to visualization of the interior of the mouth during intubation, unlike the shaft laryngoscopes used in medicine and which only allowed blind intubation. The laryngoscope of patent application EP-A-1285623 is made up of a single body formed by a first straight section and a curved section after the straight section, this last section not reaching the area under the epiglottis, which blocks the subsequent introduction of the endotracheal tube into the trachea when the epiglottis is introduced or caught in the distal part of the laryngoscope. Said body is internally divided into two closed independent conduits, one for visualizing inside the larynx during the intubation process and the other one for introducing the endotracheal tube into the patient.

The optical conduit is internally provided with different components which allow visualizing the entry point of the endotracheal tube in the larynx once the laryngoscope is introduced in the patient's mouth. Said main components are two adjacent reflective surfaces, a first surface located at the beginning of the straight section and a second reflective surface located at the end of said curved section. The end of the optical conduit that is introduced inside the patient also has a permanent transparent sheet to prevent the entry of fluids into said conduit or also a prism located on said end to achieve better visualization of the interior of the larynx. It also has a magnifying lens located on the end of the optical conduit that remains outside the patient.

After carrying out said laryngoscope to practice, it was observed that the vision of the interior of the larynx achieved through the optical conduit was not optimal and was blocked by the large amount of secretions or fluids existing inside the mouth, therefore the laryngoscope and its elements were studied and designed such that they not only achieved optimal vision of the interior of the larynx, mainly with a greater magnification power and wide-angle view, but also incorporated a device for extracting said excretions, and particularly saliva, that blocked vision.

No type of laryngoscope is known in the state of the art having an optical system such as the one disclosed and having a device incorporated on it and used when introducing said laryngoscope for extracting the fluids existing in a patient's mouth. Aspiration probes, which are independent from the laryngoscope and are connected to a manual or electrical compressor responsible for aspirating said fluids, are currently used to extract fluids existing in the mouth.

### DESCRIPTION OF THE INVENTION

The optical light laryngoscope object of the present invention is formed by a hollow longitudinal body with a first straight section, a curved section after the straight section, and a second straight section having a smaller length than the first straight section with a separation of said internal cavity into two independent conduits. The longitudinal body may have an approximately rectangular section with rounded vertexes or its section may be circular or elliptical. Said laryngoscope has a distal end that is the first one introduced in the patient's mouth and is located at the end of the second straight section, this free end coinciding with the outlet of the intubation tube inside the mouth. In this distal area, said laryngoscope has an outlet for the two independent internal conduits, one of the conduits being used for visualization and a second one for introduction and extraction of the intubation tube, said distal part further having means for incorporating the fluid extraction device. The optical conduit entry, from where the health professional visually controls the operation of introducing the laryngoscope into the patient as well as the intubation tube or endotracheal tube into the patient's trachea from where the tube exits through the distal end of the laryngoscope, is located on the opposite end, or the proximal end, of the laryngoscope.

The laryngoscope has four surfaces demarcating the hollow interior thereof, two sides, a top surface and a bottom surface. A first side opposite the central interior separation partition demarcates the optical conduit, and a second side opposite the central interior separation partition demarcates the endotracheal conduit or conduit for introducing the intubation tube. The length of the top surface of the laryngoscope has a smaller length than the bottom one since the radius of the top surface of the curved section is smaller than the radius of the bottom surface of the same section, and because the length of the top surface of the second straight section is smaller than the length of the top surface of said section.

The main drawback resolved by the present invention is to obtain an optical light laryngoscope that allows obtaining an image that is not blocked by the secretions or fluids existing inside the mouth and with optical means allowing obtaining a clear, magnified image with a wide-angle view. A more aseptic introduction of the endotracheal tube into the trachea is achieved with regard to previous laryngoscopes.

The solution is based on a laryngoscope incorporating a device that allows partially covering the laryngoscope with a flexible sheet, attached thereto and removable, preferably by means of adherent surfaces and which once the laryngoscope is introduced in the patient's mouth, allows the extraction of contaminating fluids blocking the vision and deposited during intubation on the laryngoscope and especially on the distal end thereof. This laryngoscope with a built-in extraction device is introduced in the larynx and allows a more aseptic introduction of the endotracheal tube into the patient's trachea since this sheet prevents direct contact of the distal end of the endotracheal tube with the fluids of the mouth. An optical system for the optical conduit of the laryngoscope has further been developed in combination with said built-in fluid or secretion extraction device in the laryngoscope to allow clear, magnified visualization with a wide-angle view along the entire path of the laryngoscope inside the mouth until being located inside the larynx as well as the subsequent introduction of the endotracheal tube into the trachea.

According to that described above and to solve the drawback considered, a first aspect of the invention refers to a laryngoscope incorporating an extractor device of the fluids existing inside the mouth.

Said laryngoscope with a built-in fluid extraction device further comprises an optical system formed in one embodiment by the following elements:
- a first lens located at the beginning of the first straight section of the laryngoscope that allows increasing and transmitting the reflected image,
- a second lens located about in the middle of the first straight section to magnify and transmit the image between the first lens and the first reflective element,
- said first reflective element located at the beginning of the curved section,
- a third lens located between the first reflective element and the second reflective element to optimally obtain the transmission of the image between both reflective elements,
- said second reflective element located at the end of the curved section and supported on the same wall of the body of the laryngoscope as the first reflective element, and
- a fourth lens located at the end of the second straight section, at the distal end of the body of the laryngoscope.

In another embodiment is it also possible that the optical visualization conduit includes one reflective element or no reflective element and can be replaced by other optical means or systems.

Furthermore, the optical system can incorporate a prism or prismatic lens on the proximal part of the laryngoscope allowing medical professionals using it to be at an angled position with respect to the shaft of the straight section of the laryngoscope since it deviates vision towards one side and thus is not above the patient's head. Said prism may be rotational for the purpose of allowing different positions for the health professionals in introducing the laryngoscope with a built-in fluid extraction device.

The built-in fluid or secretion extraction device in the laryngoscope covers the top surface, the outlet or distal end and it also covers an area of the bottom surface of the laryngoscope, according to the type of adhesion, with a sheet of flexible and malleable, preferably transparent, material. This device is in direct contact with said body of the laryngoscope and with the secretions, the extraction of said fluids being possible when said sheet is extracted. The sheet forming said device is adapted to the shape of the introduced laryngoscope due to its flexibility and is bent over itself, defining two segments. The first segment has two surfaces, the bottom surface being in contact with the body of the laryngoscope and is connected to the bottom surface of the second segment through the top surface. All these connections may be continuous or discontinuous, preferably with an adhesive although rivets, dyes, notches, etc., can also be used. As previously mentioned, the laryngoscope with the fluid extraction device prevents these fluids from obstructing vision during the introduction of the laryngoscope in the patient when adhered to one of the elements of the optical system, since said device covers said distal part of the laryngoscope.

This laryngoscope with the built-in fluid extraction device allows being able to be completely lubricated, even its distal part, thus preventing the existing problem of generating damages in some of the components of the optical system.

Another problem solved by the laryngoscope with a built-in fluid extraction device is to prevent the epiglottis from being caught or stuck with the distal end of the laryngoscope while introducing it in the patient's mouth, specifically with the outlet of either of the two conduits. The built-in extraction device in the laryngoscope moves the epiglottis upwards with the part of the sheet covering the distal end of the laryngoscope, allowing a complete opening of the tracheal opening and allowing vision without obstacles and the subsequent introduction of the endotracheal tube. This problem is also resolved by means of the incorporation of the second straight section in the laryngoscope arranged after the curved section and on the side opposite the first straight section, on the distal end of the laryngoscope, extending the length of the laryngoscope and allowing it to pass under the epiglottis and moving it upwards and towards the tongue, opening the field of vision and the path of the endotracheal tube towards the trachea.

The endotracheal conduit is located to the right of the optical conduit for the purpose of being adapted to the generalized use of the endotracheal tube by health professionals.

Another problem the invention solves is to obtain a laryngoscope that allows obtaining a larger size of the image in the first lens. The section of the laryngoscope is increased from an intermediate area in the first straight section to the proximal end of the laryngoscope so that the size of said image in the display or first lens is larger.

This increased section of the proximal part of the laryngoscope allows a better hold or grip of the laryngoscope with the built-in fluid extraction device by the hand of the health professional on said proximal part.

The side of said endotracheal conduit of the laryngoscope may have different constructions facilitating the separation of the endotracheal tube in order to allow the comfortable and simple separation of the laryngoscope and the endotracheal tube once the endotracheal tube has been placed in the trachea. Said side of the laryngoscope is defined by a first section with a closed surface close to the proximal end of the laryngoscope, coinciding with the housing for the batteries, followed by a second section with an open surface for introducing and extracting the endotracheal tube, and finally a third section with a variable construction surface allowing the separation of the endotracheal tube when it is necessary, but it also allows maintaining the endotracheal tube in place inside the endotracheal conduit while introducing the laryngoscope.

The laryngoscope, also has a light system formed by a power source, a conductor and a light bulb. The power source is made of standard use extractable batteries, the housing or container of which is located on the most proximal part of the side of the laryngoscope, preferably in the first section of the side of the endotracheal conduit. The fact that the batteries are extractable allows that despite the laryngoscope is used just once, batteries for following intubations can be used. The entire container of the batters can also be extractable such that the containers rather than the batteries can be exchanged between different laryngoscopes.

Said light bulb is incorporated inside the bottom surface of the distal end of the endotracheal conduits of the laryngoscope in order to reduce the perimeter of the distal end and allow correct adhesion of the fluid extraction device to the laryngoscope, and to prevent the light bulb of the light system from hurting the patient with burns due to the heat emitted by said light bulb.

The incorporation of the light bulb in said location and its inclined position allows guiding the light emitted by the light bulb in an inclined manner upwards and therefore towards the interior of the trachea, improving the lighting of the trachea with respect to other laryngoscopes generating shadows in the lighting and therefore hindering vision.

In order to facilitate adhesion of the fluid extraction device to the laryngoscope, taking advantage of the fact that the outer perimeter thereof must be as small as possible and that the endotracheal tube must be guided upwards and towards the left in its exit from the laryngoscope in order to be introduced in the trachea, the laryngoscope has:
- A wedge-shaped thickening on the side wall of the endotracheal conduit providing a larger support surface for adhesion of the built-in fluid extraction device in the laryngoscope, and a re-routing of the endotracheal tube towards the left and towards the trachea in its exit.
- A wedge-shaped thickening on the bottom wall of the endotracheal conduit providing a larger support surface for adhesion of the built-in fluid extraction device in the laryngoscope, a housing for the light bulb, with the subsequent reduction of the distal outer perimeter of the laryngoscope, and a re-routing of the endotracheal tube upwards and towards the trachea in its exit.

It is also possible to extend the central separation partition between the optical conduit and the endotracheal conduit to the end of the laryngoscope by having an inclined top side, said inclination being parallel to the inclination of the end of the side wall of the optical conduit. This extended central partition has two functions. It acts as an additional support for the fluid extraction device and further prevents the epiglottis from moving downwards and being introduced in the conduits should the laryngoscope move once it is introduced in the patient, possibly blocking either the introduction of the endotracheal tube or the visualization of the interior of the patient. Said central partition will aid in keeping the epiglottis in lifted position.

### DESCRIPTION OF THE DRAWINGS

To facilitate the understanding of this invention, 19 figures are attached to the present patent application, the purpose of which is' to better understand the grounds on which the invention at hand is based, and for a better understanding of the description of a preferred embodiment taking into account that the nature of the figures is illustrative and non-limiting.

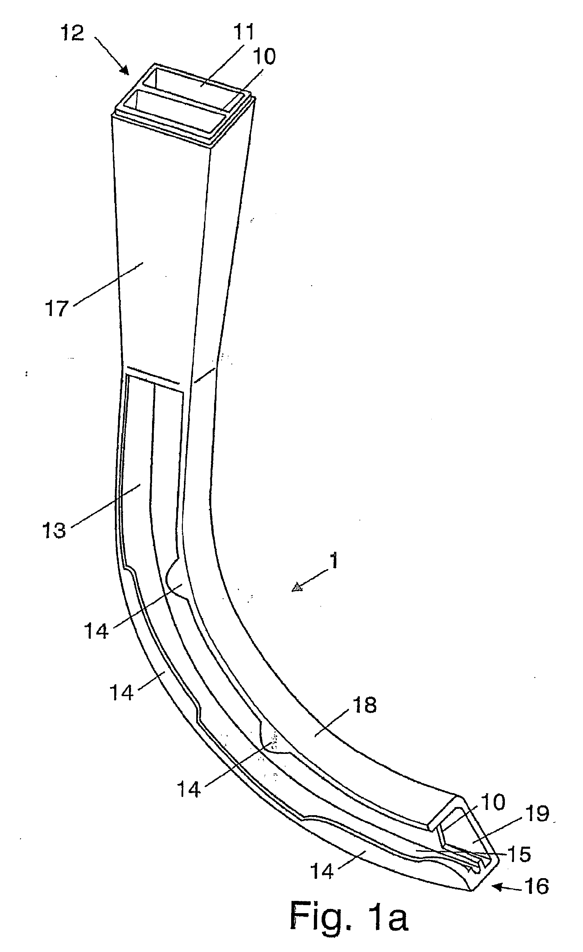

Figure 1a shows a left outer perspective view of the optical light laryngoscope of the present invention.

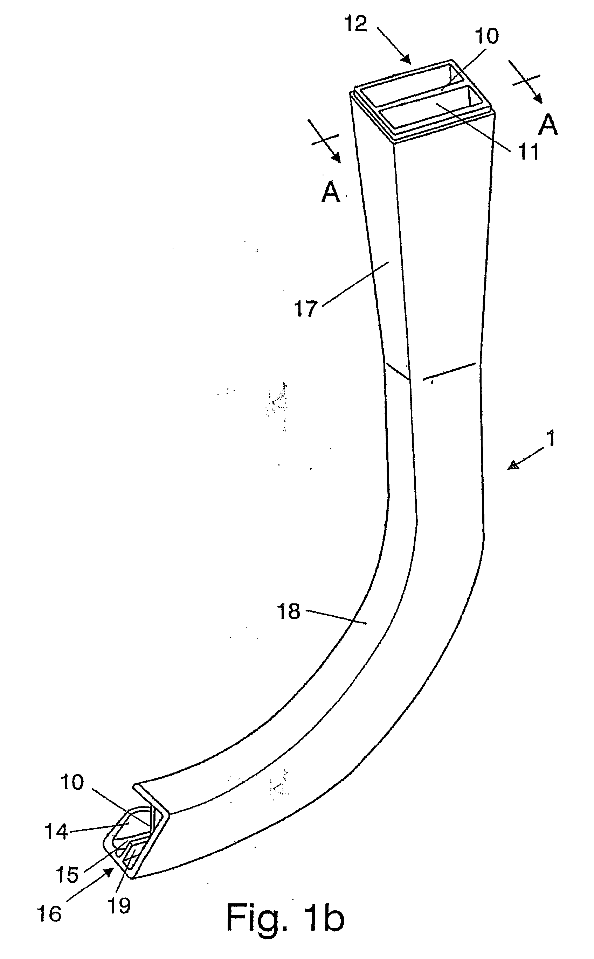

Figure 1b shows a right outer perspective view of the optical light laryngoscope of the present invention.

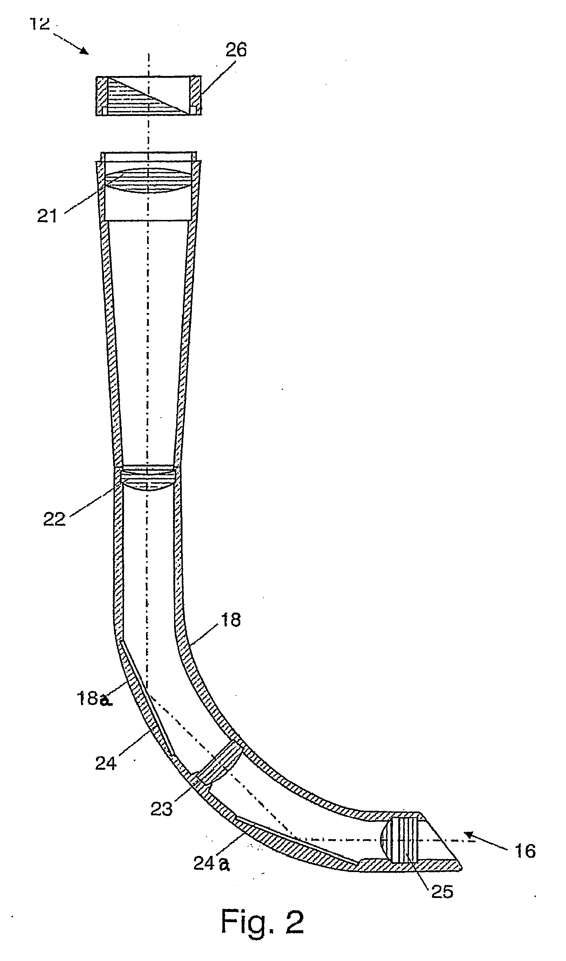

Figure 2 shows a section according to AA of Figure 1b in which the integrating elements of the vision system are observed.

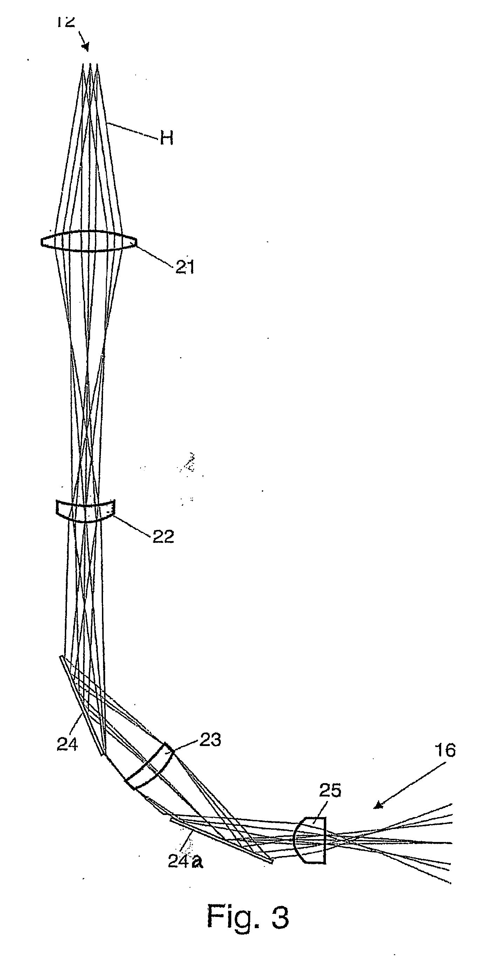

Figure 3 shows the optical system formed by the lenses and mirrors.
Figure 4a shows a profile view of the laryngoscope on the side of the endotracheal conduit with a flange in contact with and perpendicular to the bottom surface of the laryngoscope and with two projections on the ends of said flange.
Figure 4b shows a profile view of the laryngoscope on the side of the endotracheal conduit with two flanges, one in contact with and perpendicular to the bottom surface and the other one in contact with and perpendicular to the top surface, demarcating a slot or groove between them.
Figure 4c shows a profile view of the laryngoscope on the side of the endotracheal conduit with the side closed.
Figure 4d shows a profile view of the laryngoscope on the side of the endotracheal conduit with two flanges with small dimensions in contact with the top surface of the laryngoscope and two flanges with larger dimension in contact with the bottom surface.

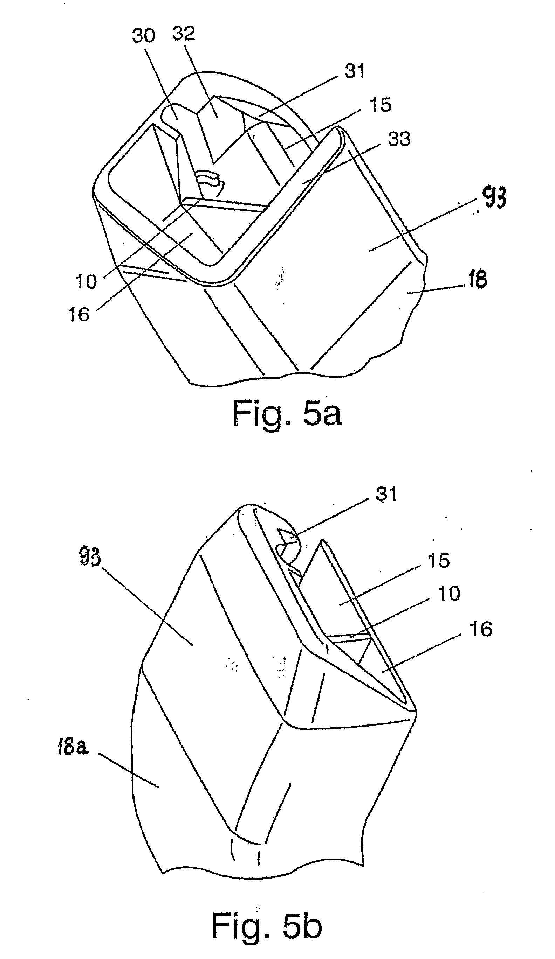

Figure 5a shows a right perspective view of the distal end of the laryngoscope with the two conduits and the housing for the light point.
Figure 5b shows a left perspective view of the distal end of the laryngoscope with the two conduits and the housing for the light point.

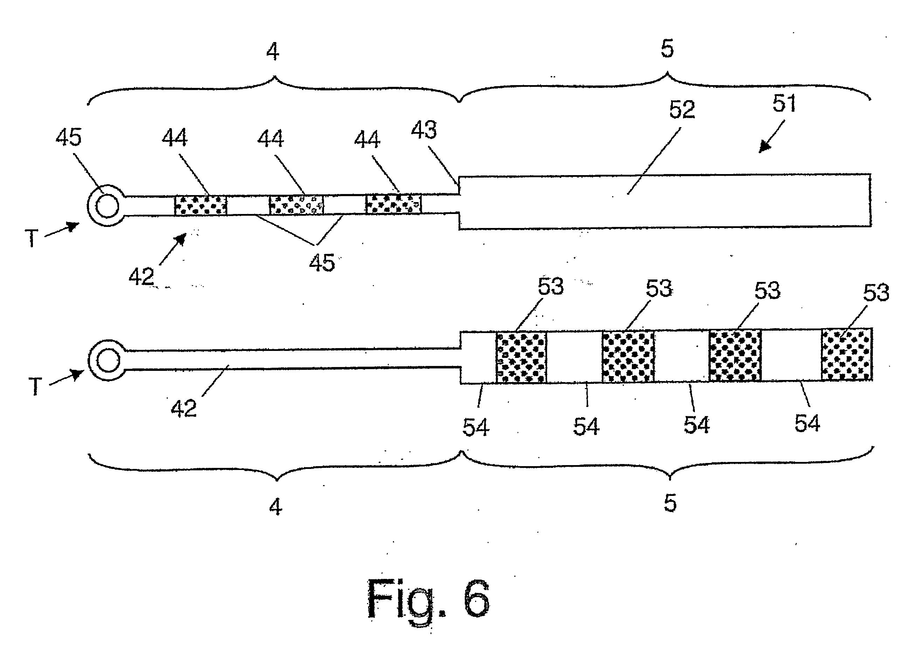

Figure 6 shows two plan views of the device for extracting oral fluids.

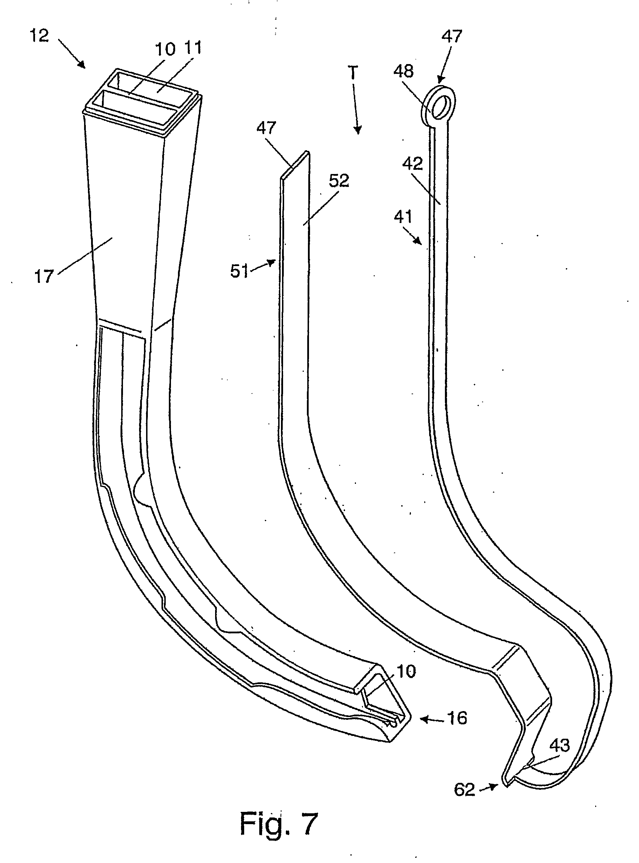

Figure 7 shows a view of the optical light laryngoscope together with the fluid extraction device.

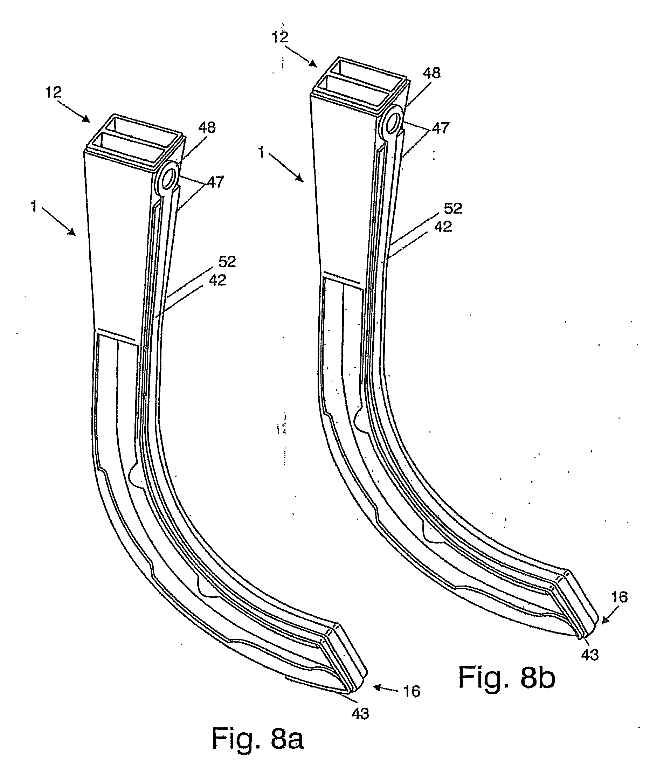

Figure 8a shows a perspective view of the fluid extraction device on the laryngoscope with the bottom end of said device adhered to the bottom part of the laryngoscope.
Figure 8b shows a perspective view of the fluid extraction device located on the laryngoscope with the bottom end of said device adhered to the bottom surface at the outlet of the endotracheal conduit.

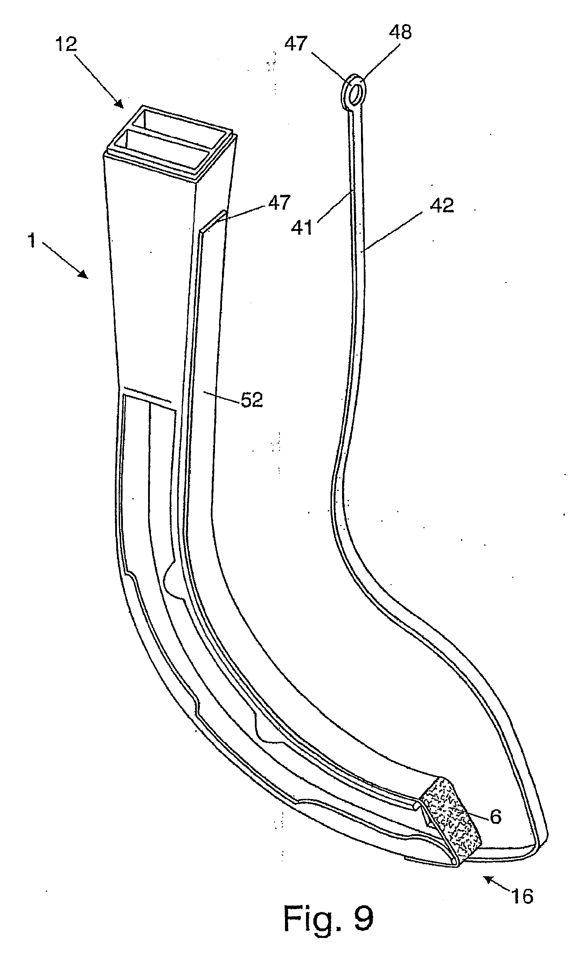

Figure 9 shows a perspective view of the device in a first phase of the extraction process thereof once the laryngoscope is introduced in the patient.

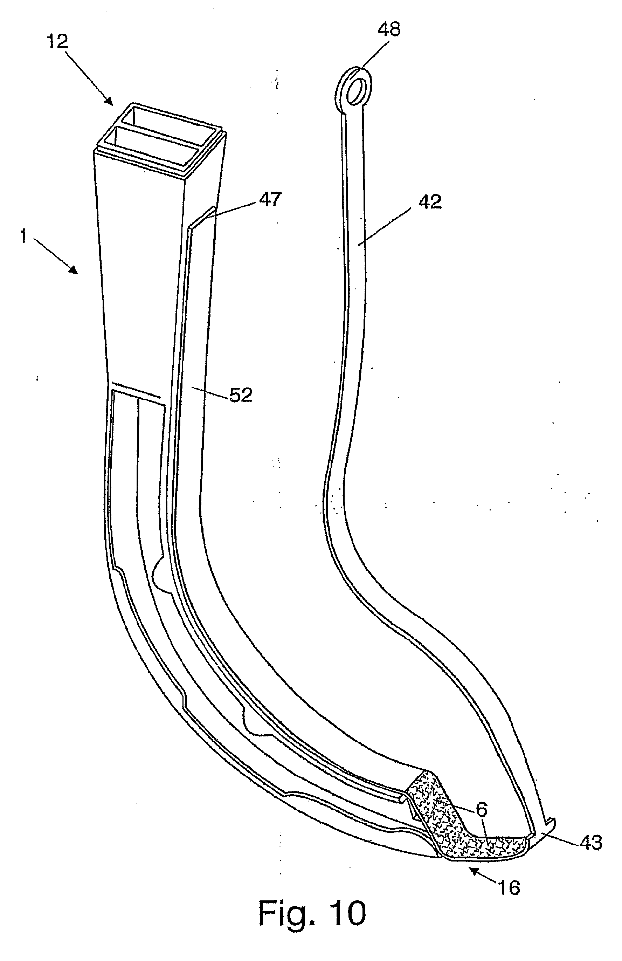

Figure 10 shows a perspective view of the device in a second phase of the extraction process thereof once the laryngoscope is introduced in the patient.

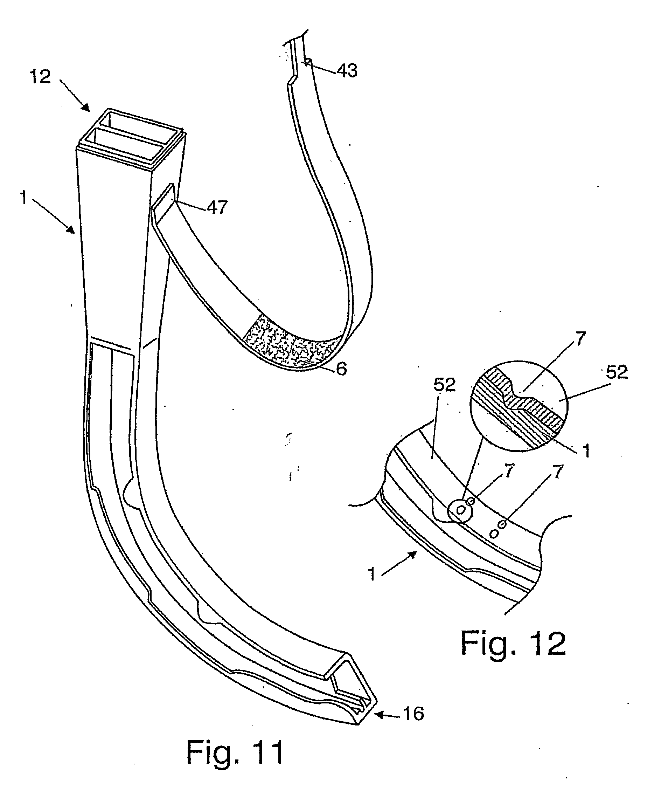

Figure 11 shows a perspective view of the device before being completely separated from the laryngoscope and completely extracted from the patient.
Figure 12 shows an alternative method of attachment between the first segment of the device and the laryngoscope.

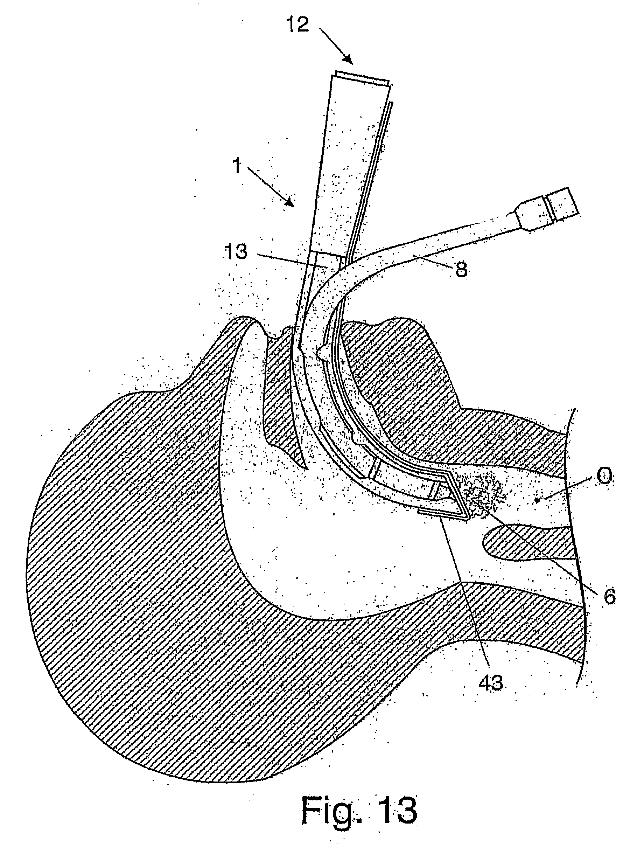

Figure 13 shows Figure 8a inside a patient with the endotracheal tube introduced in the laryngoscope.

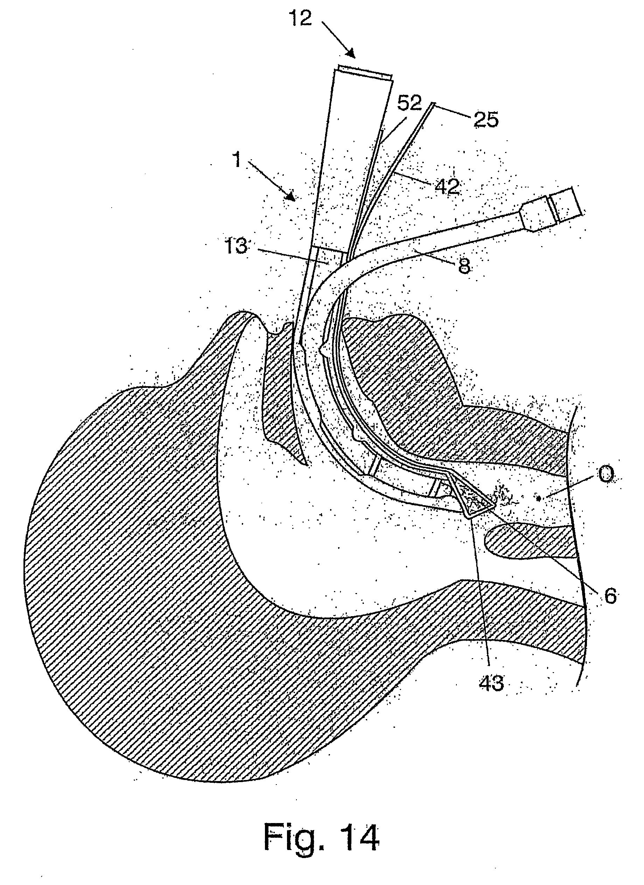

Figure 14 shows Figure 9 inside a patient with the endotracheal tube introduced in the laryngoscope.

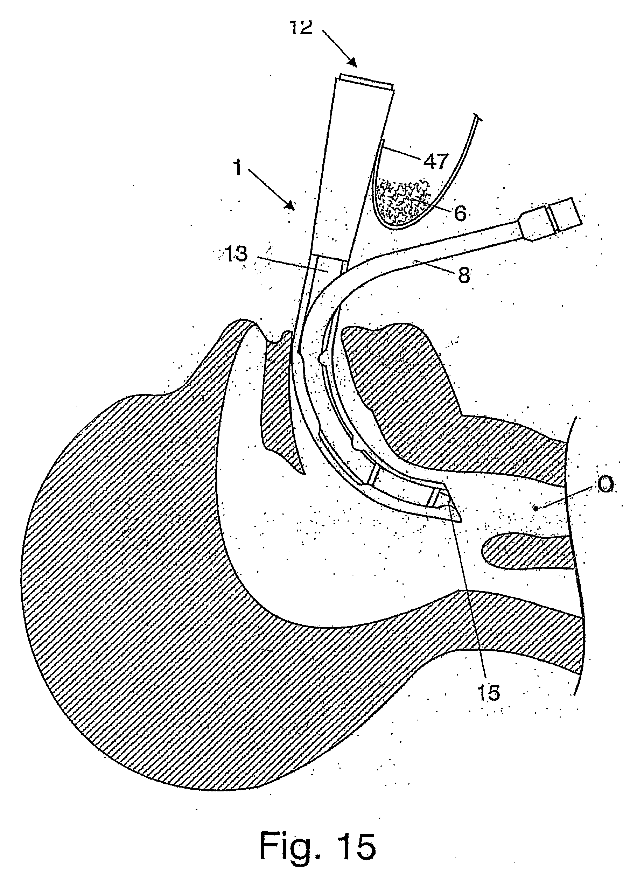

Figure 15 shows Figure 11 inside a patient with the endotracheal tube introduced in the laryngoscope.

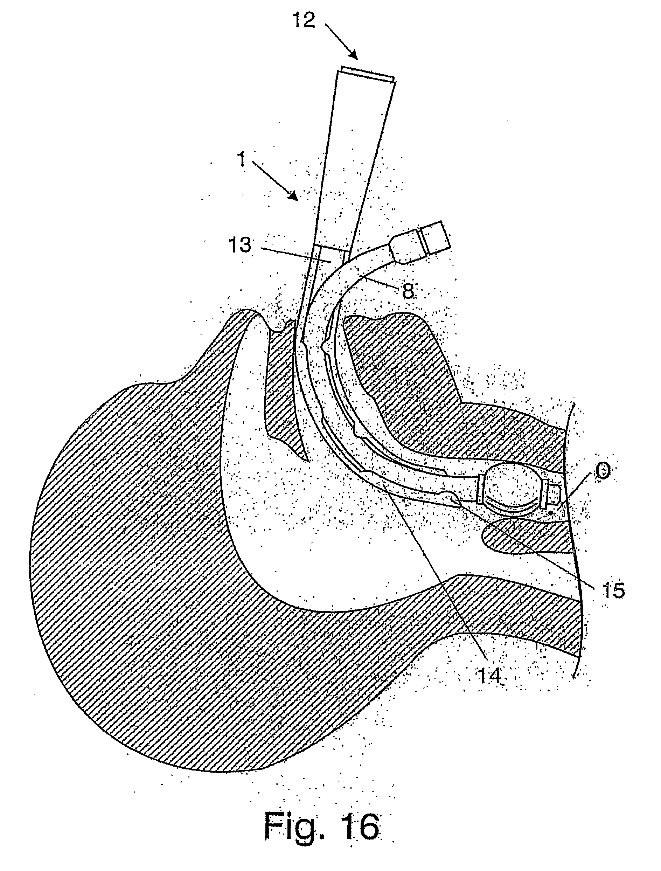

Figure 16 shows the optical light laryngoscope together with the endotracheal tube inside the trachea of a patient once the fluid extraction device is withdrawn.

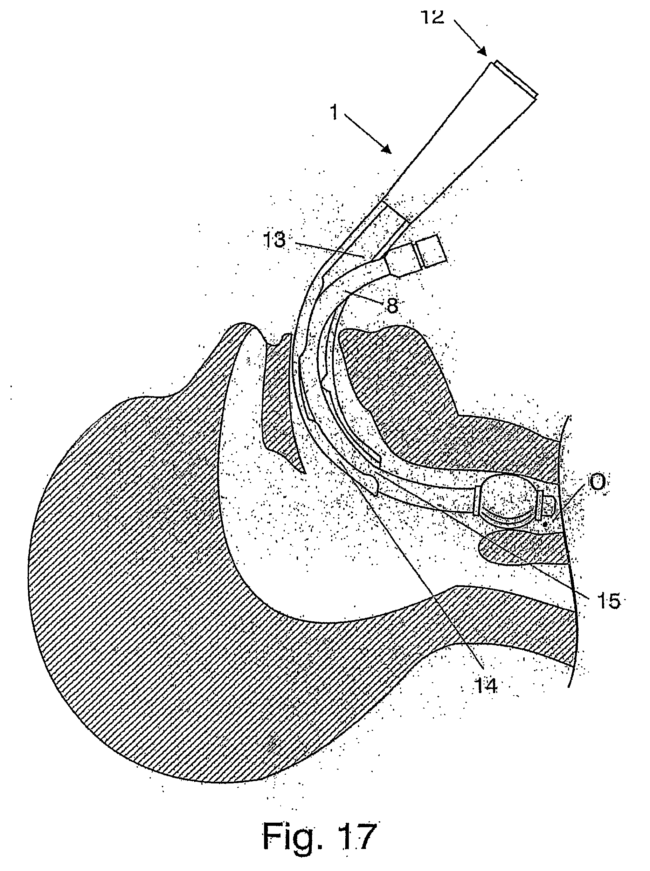

Figure 17 shows the optical light laryngoscope together with the endotracheal tube inside the trachea of a patient while the laryngoscope is extracted from the patient and laterally separated from the endotracheal tube, the endotracheal tube remaining inside the trachea.

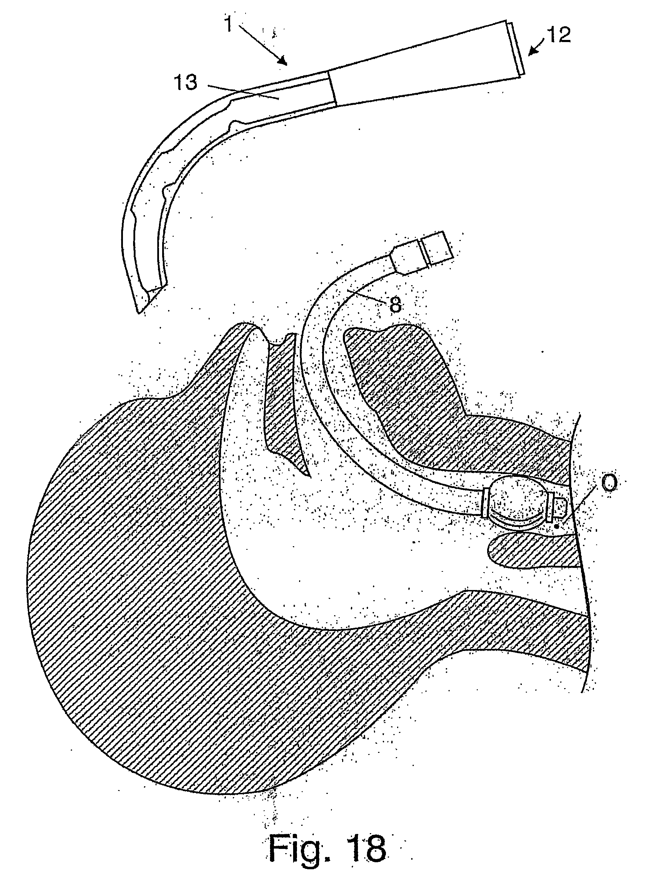

Figure 18 shows the optical light laryngoscope completely extracted from the patient and the endotracheal tube inside said patient.

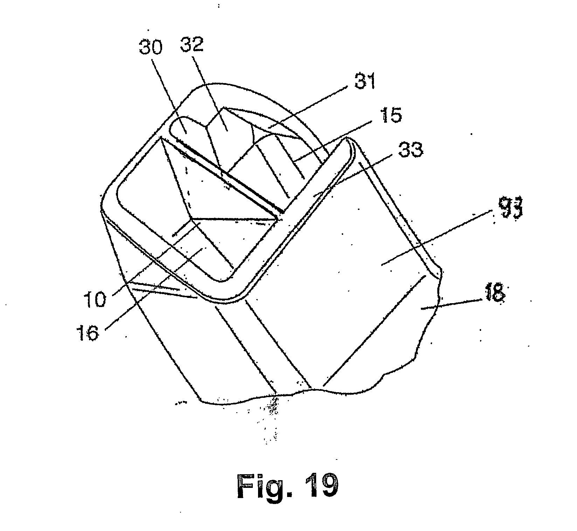

Figure 19 shows a perspective view of the distal end of the laryngoscope with the two conduits and the housing for the light point.

### DESCRIPTION OF A PREFERRED EMBODIMENT

The optical light laryngoscope 1 made up of a longitudinal, preferably prismatic body with an approximately rectangular section, has a first straight section 91 divided into a first part with a continuous section and into a second proximal part with a section growing conically from a first area 94, located between the proximal end 12 of the optical light laryngoscope and the beginning of the curved section 95 towards the proximal end 12. Said first part is followed by a curved section 92 and the latter is in turn followed by a second straight section 93, having a smaller length than the first straight section 91, the end 16 of this second straight section 93 of the laryngoscope 1 being the distal part 16 thereof coinciding with the outlet end of the intubation conduit 15 and with the end of the optical conduit 19. This distal part 16 is the part that is introduced first in the patient's mouth. The free end of the first straight section, proximal end 12, is what remains outside the patient's mouth, the curved section 92 and the second straight section 93 remaining inside the patient's mouth.

The angle formed by the shafts of the first straight section 91 and the second straight section 93 is approximately in the interval between 50º and 110º, being preferably that said angle be 90º. This angle can be found outside the first interval if the anatomical requirements of the patient so require this. Said angle depends on the laryngeal anatomy of the patient and can be affected by age, weight, height, constitution, sex and diseases suffered, among other factors.

The laryngoscope has four surfaces demarcating the hollow interior thereof, two side surfaces, a top surface 18 and a bottom surface 18a. A side surface demarcates together with the interior separation partition 10, the optical conduit and another side surface 14 opposite to the partition or central wall 10 demarcates the endotracheal conduit 15 for introducing the intubation tube 8, both conduits being parallel. The length of the top surface 18 of the laryngoscope is smaller than the length of the bottom 18a due to the fact that the radius of the top surface 18 of the curved section is smaller than the radius of the bottom surface 18a of the same section. The distal end 16 further ends with an inclination defined due to a longer length of the bottom surface 18a with respect to the top surface 18 on the second straight section 93.

The side of the endotracheal conduit is separated into three different sections. A first section 17 begins on the proximal end 12 where the housing for the power source or battery or batteries is located and extends to a first point 94 located on the first straight section 91 and before the curved section 92 begins. Said batteries provide power to the light bulb (not shown) located on the distal end 16 in the housing 30 designed to house the light bulb. The proximal part and side of the beginning of the first straight section of the laryngoscope have been used to house the batteries so as to create a housing 17 including the electrical means for the insertion thereof. The power transmission means are preferably located in the intersection between the partition or central wall 10 and the bottom surface 18a of the laryngoscope, inside the endotracheal conduit, from said housing 17 for the batteries to the cavity 30 for the light bulb. Said housing 17 for the batteries is protected by a removable cover. There can be two variants for this housing 17:
- One in which the batteries are removable such that once the laryngoscope is used, the batteries are removed in order to recycle the laryngoscope and the batteries are reusable in another laryngoscope.
- One in which the housing 17 itself is removable, such that it will not be necessary to remove the batteries but only the entire housing 17 as many times needed. The batteries may further be extractable.

An open second side section 13 for the side introduction and entry of the endotracheal tube 8 into the endotracheal conduit begins after the housing 17 for the batteries, from said first point 94 located on the first straight section 91 and before the beginning of the curved section 92 to a second point 95 located at the beginning of the curved section 92. Said opening 13 allows introducing the endotracheal tube 8 with no problems, i.e. it allows introducing the endotracheal tube 8 without compression thereof.

The third section of the side of the laryngoscope can have different configurations and extends from the end of the opening 13 located at the beginning 95 of the curved section 92 for introducing the endotracheal tube 8 to the distal end 16 of the laryngoscope.

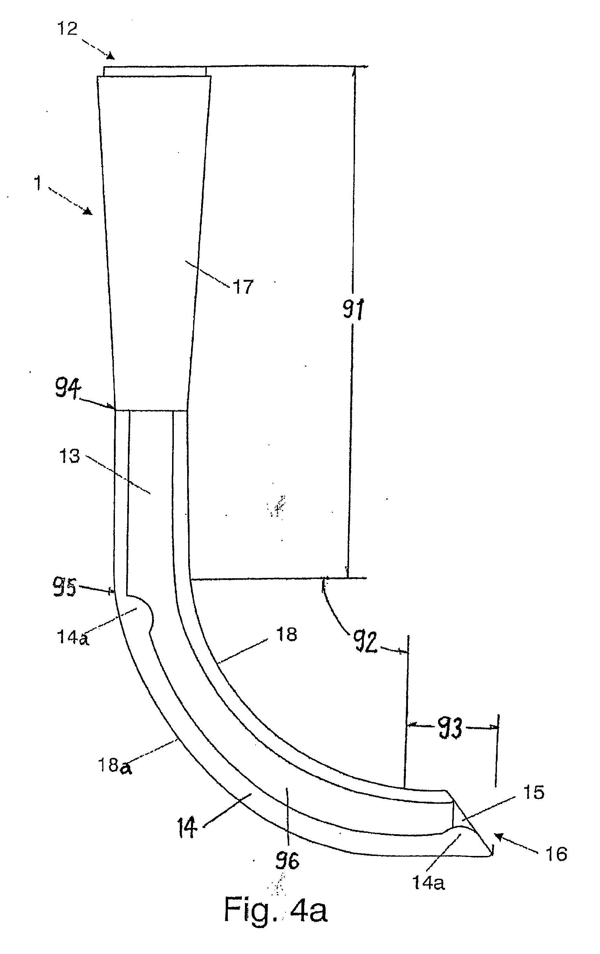

The preferred configuration of this last section of the side of the endotracheal conduit will be the one shown in Figure 4a, which shows a flange 14 that is attached and perpendicular to the bottom surface 18a of the laryngoscope, which spans the entire perimeter of the endotracheal tube from the end of said opening 13 to the distal end 16 of the laryngoscope 1, and defining a cavity 96 between said flange and the top surface 18 of the laryngoscope. The flange 14 has two projections 14a on both ends such that the cavity 96 is reduced with the top surface 18, preventing the exit of the endotracheal tube 8 unless it is compressed. The width of the cavity 96 existing between the flange 14 and the top surface 18 is larger than the cavity between the projection 14a of the flange 14 and the top surface 18.

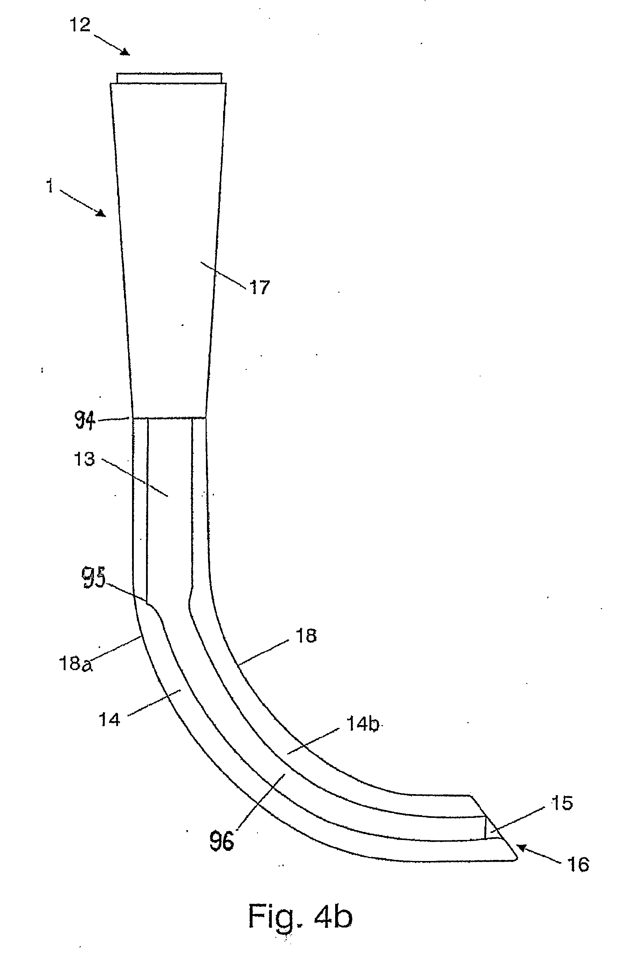

A second configuration, shown in Figure 4b, has from the end of the opening 13 to the distal end of the laryngoscope a flange 14b attached and perpendicular to the top surface of the laryngoscope and a flange 14 attached and perpendicular to the bottom surface of the laryngoscope, defining a cavity 96 between both flanges which has less width than the diameter of the endotracheal tube 8, therefore after initial compression of the tube 8 it is possible to extract it from inside the endotracheal conduit.

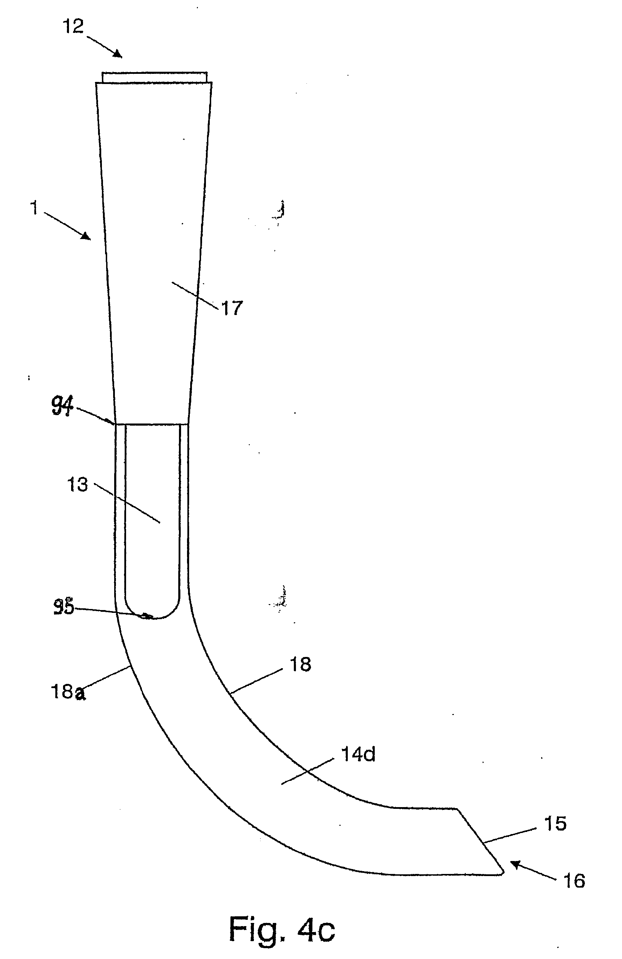

A third proposal for the configuration of the third section of the side of the endotracheal conduit, Figure 4c, consists of the latter being closed by a wall 14d.

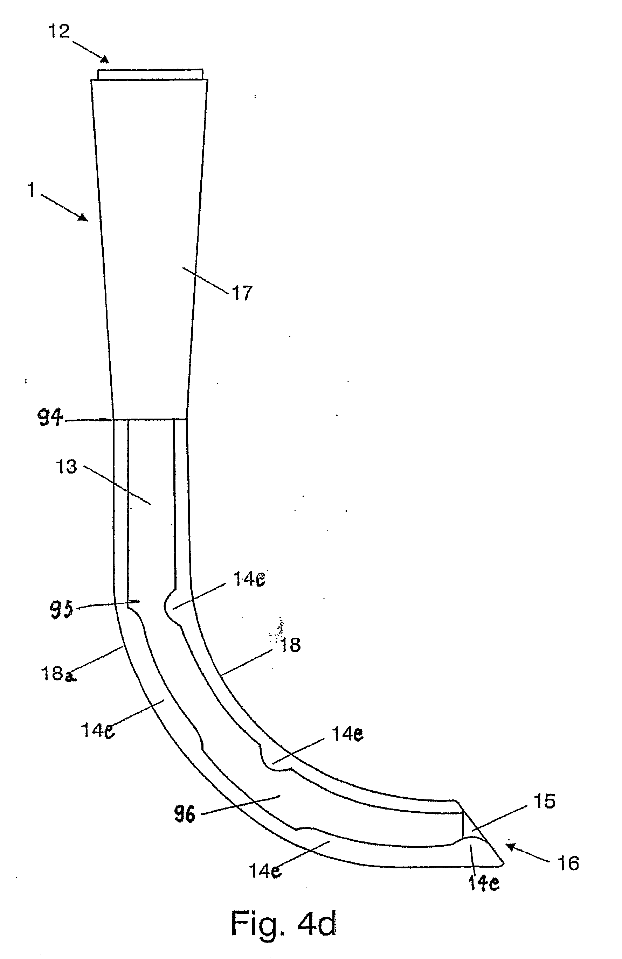

Another possible configuration is the arrangement of flanges 14a at the points in which the tube 8 is supported during its introduction in the endotracheal conduit 15. One example of said construction can be seen in Figure 4d.
The advantage of the options with flanges (Figures 4a and 4d) over the option of the cavity 96 through the entire length of the laryngoscope (4c) is that it allows a simpler lateral extraction and separation of the tube 8 since it is not necessary to compress it during the entire run during its side extraction from the laryngoscope 1. The tube 8 is held in place with the flanges, preventing the endotracheal conduit 15 from coming out and when it is extracted, there are certain compression flanges, preferably a flange 14a at the beginning of the second section 95 of the side of the endotracheal conduit 15, and another flange 14a on the distal end 16. It is possible to design other flange arrangement combinations in addition to those shown with a larger or smaller separation between them.

Once the endotracheal tube 8 is introduced in the endotracheal conduit through the opening 13 of said tube 8, it slides inside the laryngoscope 1 parallel to the optical conduit to the distal end 16 of the laryngoscope.

Said optical conduit is used for visual control of the process of introducing the laryngoscope 1 in the patient and subsequently for checking the correct introduction of the intubation tube 8 in the patient's trachea. In order to achieve that this introduction process is as fast and safe as possible, the laryngoscope has a typical construction of its optical system, being formed in one embodiment by at least four lenses and two reflective elements, as well as an additional prism located on the proximal end.

The first lens 21 with its two convex surfaces is located at the beginning of the first straight section 91 of the laryngoscope, and its function is, in combination with a second lens 22, to magnify and transmit the image reaching it reflected from the distal end 16 of the laryngoscope. The second lens 22 having a convex surface and a concave surface, an located in the first straight section 91 before the beginning of the curved section 92, has functions, in combination with the first lens 21, of magnifying and transmitting the image between the first reflective element 24 and said first lens 21. After this lens 22 there is a first reflective element, preferably a plane first-surface mirror 24, almost at the beginning of the curved section 92 and supported on the bottom surface 18a of the laryngoscope inside the optical conduit. The third lens 23, with one concave surface and the opposite surface convex, is located between the two reflective surfaces 24, 24a, and its function is to transmit the image between the two reflective elements 24, 24a. The second reflective element 24a is located almost before the end of the curved section 92, supported on the bottom surface 18a of the laryngoscope 1 inside the optical conduit and, like the first reflective element 24, is preferably a plane first-surface mirror. The fourth preferably prismatic lens 25 has a convex surface and the opposite surface is planar, with a wide-angle function and the function of changing the direction of the image located at the beginning of the second straight section 93.

It is obvious that said optical system can be replaced with another one containing one or no reflective surface.

For visualization at the entry of the laryngoscope to be greater and in order to be able to introduce a larger display, first convex concave lens 21, the section of the laryngoscope has an increase in its section from almost a point 94 located in the first straight section 91 and before the beginning of the curved section 92 to the proximal end 12 of said straight section 91.

The laryngoscope can optionally incorporate in a removable and rotational manner on its proximal end 12, a prismatic end 26 with both surfaces being planar in order to achieve an ergonomic position for the health professionals while using the laryngoscope, therefore it is not necessary to be above the head of the patient to see the reflected image.

The distal end 16 of the laryngoscope has been modified so as to allow the incorporation of the fluid extraction device while at the same time it achieves guiding the endotracheal tube 8 to the exit of the endotracheal conduit 15 towards the trachea "O" and to also allow incorporating a light bulb in the thickness of the bottom surface 18a, reducing the outer perimeter of the distal end of the laryngoscope with a built-in extraction device and being able to guide the light beam of the light bulb towards the trachea "O". The endotracheal conduit 15 in the second straight section 93 of the laryngoscope has its bottom surface 32 and side surface 31 thickened. The bottom surface 32 is thickened, being lifted and determining a housing 30 for the light bulb and the side surface 31 is inclined and thickened towards the interior of the laryngoscope on its distal end, both inclinations allowing the correct guiding of the tube 8 towards the trachea "O". Said inclined surfaces define wedges having a larger surface than the rest of the contour 33 of the distal end 16, allowing adhesion of the built-in fluid extraction device in the laryngoscope by increasing the contact surface between the laryngoscope and said device.

The central separation partition 10 between the optical conduit 19 and the endotracheal conduit 15 can also be extended to the end of the laryngoscope by having its top side inclined, said inclination being parallel to the inclination of the end of the side wall of the optical conduit 19. This extended central partition 10 has two functions, acting as an additional support for the fluid extraction device, and additionally preventing the epiglottis from dropping down and being introduced in the conduits in the event that said laryngoscope moves once the laryngoscope is introduced in the patient, possibly blocking the introduction of the endotracheal tube or blocking visualization of the inside of the patient. Said central partition 10 will aid in keeping the epiglottis raised.

The built-in oral fluid extraction device in the laryngoscope is made up of a sheet of preferably transparent flexible and malleable material T made up of two segments 4, 5. Said segments are attached in their extended form by a bending surface 43 and have two surfaces each.

In order to incorporate the fluid extraction device in the laryngoscope described, the bottom surface 51 of the first segment 5 is in contact with the top surface of the body of the laryngoscope 18 and the top surface 52 of said first segment 5 is in contact with the bottom surface 41 of the second segment 4.

The contact between the different surfaces can be carried out by means of different non-permanent attachment means such as an adhesive or notches. Said attachment means can be arranged on the different surfaces in a constant manner, i.e. the attachment means are arranged along the entire contact surface, or in an intercalated manner, i.e. the attachment means are arranged in intervals along the contact surface.

The bottom surface 51 of the first segment 5 is in contact with the top surface of the body of the laryngoscope 18, preferably through adhesive areas 53 intercalated in said bottom surface 51 of said first segment 5 with other non-adhesive surfaces 54.

The top surface 52 of said first segment 5 is in contact with the bottom surface 41 of the second segment 4, preferably through adhesive areas 44 intercalated with non-adhesive surfaces 45 on said bottom surface 41 of said second segment 4.

Once the sheet T is bent along its bend line 43, the device has two ends, a top end 47 and another bottom end 62 coinciding with the bend line 43. The bottom end 62 is located on the bottom surface 18a and close to the distal end 16 of the laryngoscope 1 and after passing through said distal end 16 of the laryngoscope 1, covering the outlet of the optical conduit 19 and the endotracheal conduit 15, it extends to a point located on the top surface 18 of the laryngoscope 1 and close to the proximal end 12 of the first straight section, the top end 47 of the sheet T being located there. The top end 47 of the sheet T, formed by the two ends of the first segment 5 and second segment 4, has a fixing means 48, preferably a ring, as an extension of the second segment 4.

A variant of the previous option for arranging the sheet T is to make the bend line 43 to coincide with the distal end 16 of the bottom surface 18a, such that the fold line (43) of the sheet (T) coincides with the attachment edge between the bottom surface (18a) and the distal end (16). The larger support surface provided by the side wedge 31 and the bottom wedge 32 located on the distal end 16 of the laryngoscope at the outlet of the endotracheal conduit 15 is thus used.

The bottom end 62 of the sheet T must preferably be located in a space where the adhesion of the sheet of the first segment 5 to the bottom surface 18a of the body of the laryngoscope 1 is assured, while the top end 47 of the sheet T must preferably be located between the middle area of the first straight section of the laryngoscope 1 and the proximal end 12 thereof.

The previous arrangement is recommended for safety reasons since it allows visually controlling the two segments of the sheet T. Should one of the segments break during the extraction of the device, the situation of the other segment can always be controlled because the two ends are always visible from outside the mouth, thus preventing any fragments of the device from being introduced inside the larynx.

The width of the second segment 4 is preferably less than the width of the first segment 5 so as to allow both segments from being easily released during their use, since the contact area between them is smaller than the area existing between the first segment 5 and the body of the laryngoscope 1. The second segment 4 having a smaller width will preferably be centered with respect to the axis of longitudinal symmetry of the sheet T.

The incorporation of the device T in the laryngoscope 1 consists of first placing the top surface 52 of the first segment 5 in contact with the bottom surface 41 of the second segment 4 by means of any of the previously mentioned means, adhesion or notches. Once both surfaces of the two segments have been placed in contact, the bottom surface 51 of the first segment 5 will be placed in contact with the body of the laryngoscope 1, the laryngoscope thus being ready for use.

Once the laryngoscope incorporates the device, the assembly of the laryngoscope with the endotracheal tube 8 preloaded in the endotracheal conduit 15 is then introduced in the mouth of the patient, first introducing the distal end 16 of the laryngoscope 1.

Prior to this operation it is possible to lubricate all the surfaces of the laryngoscope to facilitate the introduction thereof in the mouth, even its distal end 16 can be lubricated with the risk that the lubricant is introduced in the optical conduit 19 of the laryngoscope 1, which would hinder vision through the optical system, since it has the fluid extraction lamina covering the entire distal end thereof.

When introducing the laryngoscope in the larynx, the fluid extraction device pushes the epiglottis upwards, the laryngoscope sliding along its bottom part and thus being placed in its proper position opposite to the trachea, allowing perfect vision of the opening of the trachea in order to be able to see how the endotracheal tube 8 is introduced in it.

When the introduction of the laryngoscope into the larynx ends, the accumulated fluids, saliva 6 and other fluids, will be deposited on the sheet T and particularly on the top surface 52 of the first segment and the top surface 42 of the second segment 2, and when the sheet T with the fluids 6 accumulated thereon is removed, correct visualization and a more aseptic introduction of the endotracheal tube are allowed.

To remove said fluids, it is enough to pull on the ring 48, which will cause the bottom surface 41 of the second segment 4 to detach from the top surface 52 of the first segment 5, since the attachment between these two surfaces is weaker than the attachment between the surface 51 and the body of the laryngoscope 1, until reaching the bend line 43. By continuing to pull on said tab 48, an inverted C or inverted wave movement is generated which will envelope the fluids 6 deposited on the top surface 52 of the first segment 5 as the bottom surface 51 of the first segment is separated from the body of the laryngoscope 1 until reaching the top end of the sheet 47 where it will completely separate. Therefore the extraction device T of the laryngoscope 1 can be withdrawn with the accumulated fluids 6. As can be deduced from the foregoing, the functions of the device are:
- Allowing lubrication of the distal end 16 of the laryngoscope 1 without staining or damaging the optics.
- Preventing the different existing fluids from staining or contaminating the endotracheal tube 8.
- Lifting the epiglottis while introducing the laryngoscope 1 in the larynx.
- Extracting the existing fluids 6 inside the mouth at the time of intubation.
- Allowing a more aseptic introduction of the endotracheal tube 8 in the trachea O.

This enveloping movement for extracting the inverted C-shaped sheet T also allows the partial extraction of other fluids inside the larynx and which had not been deposited on the sheet T.

The laryngoscope 1 can thus be introduced with perfect visualization of the interior of the trachea "O" and better aseptic conditions of the endotracheal tube in the moment of intubation, to subsequently withdraw said laryngoscope 1 laterally, maintaining the endotracheal tube 8 inside the trachea "O".

## Claims

1. An optical light laryngoscope (1) with a built-in fluid extraction device of the type made up of a longitudinal body with a first straight section (91) and a curved section (92) after the first straight section with a proximal end (12) coinciding with the free end of the first straight section (91) and a distal end (16) on the opposite end of the proximal end (12), said body being internally divided into two independent conduits (15, 19) separated by a central partition (10), a first conduit (15) used for introducing an endotracheal tube (8) and a second conduit (11, 19) used for visualizing the interior of the patient, both conduits being limited along their entire extension by a top surface (18) and a bottom surface (18a), **characterized in that** said laryngoscope comprises a built-in fluid extraction device and an optical visualization system integrated in the optical conduit.

2. An optical light laryngoscope (1) with a built-in fluid extraction device, **characterized in that** the optical conduit (11, 19) internally has at least two reflective elements (24, 24a).

3. A laryngoscope according to claims 1 and 2, **characterized in that** said oral fluid extraction device is made up of a sheet (T) of flexible and malleable material folded over itself according to a bend line (43) and determining two removable segments, a first removable segment (5) the bottom surface (51) of which is in direct contact with the laryngoscope (1), and a second removable segment (4) in direct contact through its bottom surface (41) with the top surface (52) of the first segment (5), determining a top end (47) and a bottom end (62) coinciding with the bend line (43) when one segment is bent over the other.

4. A laryngoscope according to claim 3, **characterized in that** the direct contact between the bottom surface (51) of the first removable segment (5) and the laryngoscope (1) is assured by means of non-permanent attachment means.

5. A laryngoscope according to claim 4, **characterized in that** said attachment means are notches made on the surface of the first removable segment (5) when it is on the laryngoscope (1).

6. A laryngoscope according to claims 3 to 5, **characterized in that** the bottom surface (51) of the first segment (5) is in contact with the laryngoscope (1) from the rear part (18a) of the distal end (16) of the laryngoscope (1), covering said distal end (16), to a point located on the top surface (18) of the laryngoscope (1) between said distal end (16) and the proximal end (12).

7. A laryngoscope according to claims 3 to 5, **characterized in that** the bottom surface (51) of the first segment (5) is in contact with the laryngoscope (1) from the distal end (16) of the bottom surface (18a), coinciding with the bend line (43) of the sheet (T) with the attachment edge between the bottom surface (18a) and the distal end (16), covering said distal end (16), to a point located on the top surface (18) of the laryngoscope (1) between said distal end (16) and the proximal end (12).

8. A laryngoscope according to claim 3, **characterized in that** the contact between the bottom surface (41) of the second removable segment (4) and the top surface (52) of the first removable segment (5) is carried out by means of non-permanent attachment areas (44) located on the bottom surface (41) of the second removable segment (4).

9. A laryngoscope according to claims 4 and 8, **characterized in that** the attachment areas are intercalated.

10. A laryngoscope according to claims 4 and 8, **characterized in that** the attachment areas are continuous.

11. A laryngoscope according to claims 4 and 8, **characterized in that** said attachment means are an adhesive.

12. A laryngoscope according to claim 8, **characterized in that** said attachment means are notches made on the surface of the first removable segment (5) when the latter is located on the body of the medical material (1).

13. A laryngoscope according to claim 3, **characterized in that** the sheet of flexible and malleable material (T) is transparent.

14. A laryngoscope according to the previous claims, **characterized in that** the width of the first segment (5) of the sheet (T) is greater than the width of the second segment (4).

15. A laryngoscope according to the previous claims, **characterized in that** the second segment (4) has on its free end (47), opposite to the attachment end (43) with the first segment (5), fixing means (48) not adhered to the first segment.

16. A laryngoscope according to claim 15, **characterized in that** said fixing means are a ring (48).

17. A laryngoscope according to claim 15, **characterized in that** said fixing means are a tab.

18. A laryngoscope according to the previous claims, **characterized in that** it is for a single use.

19. A laryngoscope according to claims 1 and 2, **characterized in that** the section of the laryngoscope increases conically from a first area (94) located in the first straight section (91) and towards the proximal end (12) of the laryngoscope in order to achieve introducing a first lens (21) having a larger dimension and thus magnifying the size of the reflected image and improving the grip of the laryngoscope by the health professionals.

20. A laryngoscope according to claims 1 and 2, having a side opposite to the central partition (10) demarcating the endotracheal conduit (15), **characterized in that** the side of the endotracheal conduit is open (13) for the lateral introduction of the endotracheal tube (8) from a first point (94) located on the first straight section (91) before the beginning of the curved section (92) until a second point (95) located at the beginning of the curved section.

21. A laryngoscope according to claim 20, **characterized in that** the side of the endotracheal conduit is completely closed (14d) from said second point (95) at the beginning of the curved section (92) to the distal end of the laryngoscope (16), thus joining the top surface (18) of the laryngoscope and the bottom surface (18a) thereof.

22. A laryngoscope according to claim 20, **characterized in that** the side of the endotracheal conduit has, from said second point (95), at the beginning of the curved section (92), to the distal end (16) of the laryngoscope, a flange (14b) attached and perpendicular to the top surface (18) of the laryngoscope, and a flange (14) attached and perpendicular to the bottom surface (18a) of the laryngoscope, determining a cavity (96) between both flanges (14, 14b).

23. A laryngoscope according to claim 20, **characterized in that** the side of the endotracheal conduit has, from said second point (95), at the beginning of the curved section, to the distal end (16) of the laryngoscope, a flange (14) attached and perpendicular to the bottom surface (18a) of the laryngoscope, determining a cavity (96) between said flange (14) and the top surface (18) of the laryngoscope to the distal end (16) thereof.

24. A laryngoscope according to claim 23, **characterized in that** the beginning of the flange (14) on the distal end (16) of the laryngoscope and the end of said flange (14), furthest from the distal end (16) of the laryngoscope, have a projection (14a) reducing the width of the cavity (96).

25. A laryngoscope according to claims 23 and 24, **characterized in that** the width of the cavity (96) existing between the flange (14) and the top surface (18) is greater than the width of the cavity (96) between the projection (14a) of the flange (14) and the top surface (18).

26. A laryngoscope according to claim 21, **characterized in that** the side of the endotracheal conduit has, from said second point (95), at the beginning of the curved section (92), to the distal end (16) of the laryngoscope, several flanges (14e) attached and perpendicular to the bottom surface (18a) of the laryngoscope and flanges (14e) attached and perpendicular to the top surface (18) thereof, determining a cavity (96) between them.

27. A laryngoscope according to claims 22 and 26, **characterized in that** said cavity (96) has a width that is less than the diameter of the endotracheal tube (8).

28. A laryngoscope according to claims 1 and 2, **characterized in that** it incorporates a second straight section (93) after the curved section (92).

29. A laryngoscope according to claims 1, 2 and 28, **characterized in that** said optical visualization system integrated in the optical conduit (11, 19) is internally made up of:
- a first lens (21) located at the beginning of the first straight section (91) of the laryngoscope (1) that allows increasing and transmitting the reflected image,
- a second lens (22) located in the first straight section (91) between the proximal end (12) and the beginning (95) of the curved section (92) to magnify and transmit the image between the first lens (21) and the first reflective element (24),
- said first reflective element (24) located at the beginning (95) of the curved section (92), supported on the bottom surface (18a),
- a third lens (23) located between the first reflective element (24) and the second reflective element (24a) to optimally obtain the transmission of the image between both reflective elements (24, 24a),
- said second reflective element (24a) located at the end of the curved section (92) and supported on the same wall of the body of the laryngoscope as the first reflective element (24), supported on its bottom surface (18a), and
- a fourth lens (25) located at the beginning of the second straight section (93), on the distal end (16) of the body of the laryngoscope.

30. A laryngoscope according to claims 1 and 2, **characterized in that** it removable and rotationally incorporates on the proximal end (12) of the laryngoscope a prismatic lens (26) with both surfaces being planar so as to achieve an ergonomic position for the health professionals while using the laryngoscope.

31. A laryngoscope according to claim 29, **characterized in that** the first lens (21) has its two surfaces convex with functions of magnifying and transmitting the image together with the second lens (22).

32. A laryngoscope according to claim 29, **characterized in that** the second lens (22) has one concave surface and the opposite surface convex with functions of magnifying the image together with the first lens (21) and transmitting the image between the first reflective surface (24) and the first lens (21).

33. A laryngoscope according to claim 29, **characterized in that** the first reflective element (24) is a first-surface mirror.

34. A laryngoscope according to claim 29, **characterized in that** the third lens (23) has a concave surface and the opposite surface convex for transmitting the image between both reflective elements (24, 24a).

35. A laryngoscope according to claim 29, **characterized in that** the second reflective element (24a) is a first-surface mirror.

36. A laryngoscope according to claim 29, **characterized in that** the fourth lens (25) is a convex prismatic lens with a wide-angle and image deviation function.

37. A laryngoscope according to claim 28, **characterized in that** the end of the second straight section (93) of the laryngoscope corresponding to the endotracheal conduit (15) has its bottom surface (32) and side surface (31) thickened and inclined upwards and inwards, respectively, defining a bottom wedge (32) and a side wedge (31), to deviate the endotracheal tube (8) towards the trachea (O) in its exit and providing a larger support surface for the fluid extraction device.

38. A laryngoscope according to claim 37, **characterized in that** the central separation partition (10) between the optical conduit (19) and the endotracheal conduit (15) extends to the end of the laryngoscope with its inclined top side, said inclination being parallel to the inclination of the end of the side wall of the optical conduit (19).

39. A laryngoscope according to claims 37 and 38, **characterized in that** the bottom wedge (32) has a housing (30) for introducing the light bulb.

40. A laryngoscope according to claim 20, **characterized in that** it has a housing for batteries (17) located on the side of the endotracheal conduit, between its proximal end (12) and said first point (94) located on the first straight section (91) and before the beginning (95) of the curved section (92).

41. A laryngoscope according to claim 40, **characterized in that** said housing (17) for the batteries is removable.

42. A laryngoscope according to claims 1 and 2, **characterized in that** the longitudinal body has a rectangular section with rounded vertexes.

43. A laryngoscope according to claims 1 and 2, **characterized in that** the longitudinal body has a circular section.

44. A laryngoscope according to claims 1 and 2, **characterized in that** the longitudinal body has an elliptical section.
